# EUROPEAN PATENT SPECIFICATION

(11) **EP 2 922 963 B1**
(45) Date of publication and mention of the grant of the patent: **31.08.2016**
(21) Application number: 13791954.4
(22) Date of filing: 06.11.2013
(51) Int. Cl.: C12P 7/18, C12N 9/02

(54) **PROCESS FOR PRODUCING ALPHA,OMEGA-DIOLS FROM C4-ALKANES OR C4-1-ALKANOLS EMPLOYING A CYP153 ALKANE HYDROXYLASE**
VERFAHREN ZUR HERSTELLUNG VON ALPHA,OMEGA-DIOLEN AUS C4-ALKANEN ODER C4-1-ALKANOLEN UNTER VERWENDUNG EINER CYP153 ALKANHYDROXYLASE
PROCÉDÉ DE PRODUCTION D'ALPHA,OMÉGA-DIOLES À PARTIR D'ALCANES EN C4 OU DE 1-ALCANOLES EN C4 EN UTILISANT UNE CYP153 ALCANE HYDROXYLASE

(30) Priority: 20.11.2012 EP 12193378
(43) Date of publication of application: 30.09.2015
(73) Proprietor: Evonik Degussa GmbH, 45128 Essen (DE)
(72) Inventor: ENGEL, Philip, 45147 Essen (DE); HAAS, Thomas, 48161 Münster (DE); PFEFFER, Jan Christoph, 63452 Hanau (DE); THUM, Oliver, 40880 Ratingen (DE); GEHRING, Christian, 45770 Marl (DE)
(86) International application number: PCT/EP2013/073107
(87) International publication number: WO 2014/079683

(56) References cited:
- WO-A1-2011/131420
- US-B1- 8 361 769
- FUJII, T. ET AL.: "Production of alpha,omega-Alkanediols Using Escherichia coli Expressing a Cytochrome P450 from Acinetobacter sp. OC4", BIOSCIENCE, BIOTECHNOLOGY, AND BIOCHEMISTRY, vol. 70, no. 6, June 2006 (2006-06), pages 1379-1385, XP008116557,
- SCHEPS, D. ET AL.: "Regioselective omega-hydroxylation of medium-chain n-alkanes and primary alcohols by CYP153 enzymes from Mycobacterium marinum and Polaromonas sp. strain JS666", ORGANIC & BIOMOLECULAR CHEMISTRY, vol. 9, no. 19, 7 October 2011 (2011-10-07), pages 6727-6733, XP002705294, cited in the application
- KOCH, D.J. ET AL.: "In Vivo Evolution of Butane Oxidation by Terminal Alkane Hydroxylases AlkB and CYP153A6", APPLIED AND ENVIRONMENTAL MICROBIOLOGY, vol. 75, no. 2, January 2009 (2009-01), pages 337-344, XP002705295,

## Description

The present invention relates to a method comprising the steps
a) providing an alkane or 1-alkanol,
b) contacting said alkane or 1-alkanol in an aqueous solution with a cytochrome P450 alkane hydroxylase from the CYP153 family and oxygen for at least 3 hours,
wherein the alkane or 1-alkanol is converted to the corresponding α,ω-diol,
and the alkane or 1-alkanol is butane, isobutane, butanol or isobutanol.

Substituted alkanes, for example alcohols, aldehydes, ketones, carboxylic acids and amines, represent of a class of industrially sought-after compounds traditionally prepared by conversion of compounds made from fossil carbon sources. In an era of increasingly limiting supplies of non-renewable fossil fuels, there is considerable interest in biotechnological processes for producing alkanes and derivates thereof starting with renewable resources, i. e. materials that are easily and, in terms of geological time scales, rapidly replenishable.

Numerous methods for converting an alkane into substituted alkane, in particular oxidised alkanes, have been reported in the prior art. Methane monooxygenase catalyse the NADH-dependent insertion of one atom of oxygen into the exceptionally stable C-H bond of methane to form methanol, the first step in the degradation of methane by methanotrophs such as *Methylosinus trichosporium* and *Methylococcus capsulatus.* The soluble methan monooxygenase typically have a broad substrate spectrum including saturated and unsaturated, linear, branched, and cyclic hydrocarbons up to about C8, as well as aromatic, heterocyclic, and chlorinated compounds (Merkx M, Kopp DA, Sazinsky MH, Blazyk JL, Müller J, Lippard SJ (2001), Angew Chem Int Ed Engl 40:2782-2807; Higgins IJ, Best DJ, Hammond RC. 1980). New findings in methane-utilizing bacteria highlight their importance in the biosphere and their commercial potential. Rubredoxin-dependent alkane monoxygenases such as the alkane monooxygenase from *Pseudomonas putida* GPo1 catalyse the oxidation of alkanes of medium chain lengths, yielding a mixture of alcohols and carboxylic acids (Grant C., Woodley, J. M, and Baganz, F (2011) Enzyme and Microbial Technology 48, 480-486).

However, whilst alkyls having two or more substitutions are essential for many uses, for example as crosslinkers in the production of industrial polymers, the degree of substitution that may be achieved using such biotechnological approaches is limited. Many systems have been reported to convert an alkane into the corresponding 1-alkanol, but few biotechnological processes exist that may be used to produce the corresponding α,ω-diol, *i.* e. an alkyl chain, wherein the two terminal carbon atoms each carry one hydroxyl group. What.is more, many oxidases tend to over oxidise the alkane substrate to the effect that unwanted aldehydes and carboxylic acids contaminate the sought-after alcohol product. Finally, the oxidation needs to be selective, meaning that preferably no alkane carbon atoms other than the terminal ones should be oxidised.

The document FUJII, T. ET AL.: "Production of alpha,omega-Alkanediols Using Escherichia coli Expressing a Cytochrome P450 from Acinetobacter sp. OC4" (BIOSCIENCE, BIOTECHNOLOGY, AND BIOCHEMISTRY, vol. 70, no. 6, June 2006, pages 1379-1385) discloses a method for converting a C₅-C₁₄ alkane or a C₆-C₁₂ 1-alkanol into to the corresponding α,ω-diol comprising contacting said alkane or 1-alkanol in an aqueous solution with a cytochrome P450 alkane hydroxylase of the CYP153 family and oxygen for 24 hours.

Further, the document SCHEPS, D. ET AL.: "Regioselective omega-hydroxylation of medium-chain n-alkanes and primary alcohols by CYP153 enzymes from Mycobacterium marinum and Polaromonas sp. strain JS666" (ORGANIC & BIOMOLECULAR CHEMISTRY, vol. 9, no. 19, 7 October 2011, pages 6727-6733) discloses a method for converting a C₅-C₁₂ alkane or a C₈-C₁₂ 1-alkanol into to the corresponding α,ω-diol comprising contacting said alkane or 1-alkanol in an aqueous solution with a cytochrome P450 alkane hydroxylase of the CYP153 family and oxygen for 4 hours.

The document KOCH, D.J. ET AL.: "In Vivo Evolution of Butane Oxidation by Terminal Alkane Hydroxylases AlkB and CYP153A6" (APPLIED AND ENVIRONMENTAL MICROBIOLOGY, vol. 75, no. 2, January 2009, pages 337-344) discloses a method for converting a C₃-C₈ into to the corresponding 1-alkanol comprising contacting said in an aqueous solution with a cytochrome P450 alkane hydroxylase of the CYP153 family and oxygen for 1 hour.

Therefore, the problem underlying the present invention is to provide a biotechnological route towards the conversion of an alkane or 1-alkanol to the corresponding α,ω-diol, wherein the degree of over oxidation is limited and/or the reaction is sufficiently specific, preferably in that carbon atoms other than the terminal carbon atoms are not substituted or substituted to a lesser extent.

The problem underlying the present invention is solved by a method comprising the steps
a) providing an alkane or 1-alkanol,
b) contacting said alkane or 1-alkanol in an aqueous solution with a cytochrome P450 alkane hydroxylase from the CYP153 family and oxygen for at least 3 hours,
wherein the alkane or 1-alkanol is converted to the corresponding α,ω-diol,
and the alkane or 1-alkanol is butane, isobutane, butanol or isobutanol.

In a first embodiment, the problem is solved by a method, wherein, in addition to the cytochrome P450 alkane hydroxylase from the CYP153 family, a ferredoxin and a ferredoxin reductase is present, wherein both the ferredoxin and the ferredoxin reductase are capable of functionally interacting with said Cytochrome P450 alkane hydroxylase from the CYP153 family.

In a second embodiment, the problem is solved by a method, wherein the cytochrome P450 alkane hydroxylase from the CYP153 family is the cytochrome P450 alkane hydroxylase from the CYP153 family from *Alcanivorax borkumensis* (access code YP_691921) or a variant thereof, the ferredoxin is the ferredoxin from *Alcanivorax borkumensis* or a variant thereof and the ferredoxin reductase is the ferredoxin reductase from *Alcanivorax borkumensis* (access code YP_691923) or a variant thereof.

In a third embodiment which is also an embodiment of the first to second embodiments, the problem is solved by a method wherein the alkane is butane or isobutane.

In a 4^{th} embodiment which is also an embodiment of the first to third embodiments, the problem is solved by a method, wherein the alkane or 1-alkanol is butanol or isobutanol.

In a 5^{th} embodiment which is also an embodiment of the first to 4^{th} embodiments, the problem is solved by a method, wherein the alkane is a gaseous alkane provided at a partial pressure of 1 to 50, preferably 1 to 20 , most preferably 1 to 10 bar.

In a 6^{th} embodiment, which is also an embodiment of the first to 5^{th} embodiments, the problem is solved by a method, wherein at least one of the enzymes selected from the group comprising the cytochrome P450 alkane hydroxylase from the CYP153 family, ferredoxin and ferredoxin reductase are provided in the form of a whole cell catalyst expressing said enzyme or enzymes.

In a 7^{th} embodiment, which is also an embodiment of the first to 6^{th} embodiments, the problem is solved by a method, wherein all of the enzymes selected from the group comprising the cytochrome P450 alkane hydroxylase from the CYP153 family, ferredoxin and ferredoxin reductase are provided in the form of a whole cell biocatalyst expressing said enzymes.

In a 8^{th} embodiment, which is also an embodiment of the first to 7^{th} embodiments, the problem is solved by a method, wherein at least one, preferably all of the enzymes selected from the group comprising the cytochrome P450 alkane hydroxylase from the CYP153 family, ferredoxin and ferredoxin reductase are recombinant and/or overexpressed, preferably overexpressed.

In a 9^{th} embodiment, which is also an embodiment of the first to 8^{th} embodiments, the problem is solved by a method, wherein the whole cell biocatalyst expresses a polypeptide from the AlkL family, preferably AlkL aus *Pseudomonas putida* (Access code CAB69081) or a variant thereof.

In a 10^{th} embodiment, which is also an embodiment of the first to 9^{th} embodiments, the problem is solved by a method, wherein the whole cell biocatalyst is a prokaryotic cell, preferably *E. coli.*

The present invention is based on the surprising finding that cytochrome P450 alkane hydroxylase from the CYP153 family may be used to convert an alkane not only to the corresponding 1-alkanol, but also to the corresponding α,ω-diol.

The present invention centers around a method comprising the step contacting butane, isobutane, butanol or isobutanol with a cytochrome P450 alkane hydroxylase from the CYP153 family. In a preferred embodiment, the term "cytochrome P450 alkane hydroxylase from the CYP153 family", as used herein, refers to a cytosolic cytochrome oxidase which is naturally part of a 3 component-system comprising not only the oxidase, but also a ferredoxin and a ferredoxin reductase, which oxidase has an alkane binding site and is capable of hydroxylating alkanes. In a more preferred embodiment, the cytochrome P450 alkane hydroxylase from the CYP153 family has at least 80, preferably 90, more preferably 95 and most preferably 99 percent amino acid sequence identity with the cytochrome P450 oxidase from *Alcanivorax borkumensis* SK2 (access code YP_691921) and has, in addition, alkane hydroxylase activity. In another preferred embodiment, the cytochrome P450 alkane hydroxylase has, in addition to said degree of amino acid sequence identity, said binding site and said alkane hydroxylase activity, the amino acid sequence motif LL(I/L)(V/I)GGNDTTRN. The data base codes cited throughout this application refer to those of the NCBI data base version online on 12^{th} October 2012.

For providing as efficient as possible a supply of electrons from the reducing agent to sustain the hydroxylase activity of the cytochrome P450 alkane hydroxylase it is preferred that the alkane hydroxylase is used in combination with a ferredoxin reductase and a ferredoxin functionally interacting with said alkane hydroxylase. The ferredoxin and ferredoxin reductase may be isolated polypeptides or may be coexpressed in case a whole cell biocatalyst is used to provide the cytochrome P450 alkane hydroxylase. Whether or not a ferredoxin reductase and/or a ferredoxin interact functionally with a cytochrome P450 alkane hydroxylase of interest may be readily determined by the person skilled in the art by monitoring whether the reducing agent is oxidised beyond background level in the presence of an alkane substrate and the three polypeptides, the latter being preferably isolated polypeptides. Alternatively, an enzyme assay described by Scheps, D., Malca, H., Hoffmann, B., Nestl, B. M, und Hauer, B. (2011) Org. Biomol. Chem., 9, 6727 may be used that indicates an increased reaction rate in case functionally interacting polypeptides are used. In a preferred embodiment, the cytochrome P450 alkane hydroxylase from the CYP 153 family, the ferredoxin and the ferredoxin reductase are from the same organism, more preferably from *Alcanivorax borkumensis* SK2, in which case the access codes are YP_691921, YP_691923 and YP_691920, respectively.

In a preferred embodiment, the term "contacting", as used herein, means bringing about direct contact between butane, isobutane, butanol or isobutanol and oxidoreductase such that the latter is able to oxidise the former. For example, the cell and the alkane or 1-alkanol may not be in different compartments separated by a membrane such as an inorganic membrane impermeable for the cell and the butane, isobutane, butanol or isobutanol. If the alkane or 1-alkanol is solid or soluble, it may simply be added to the oxidoreductase in an aqueous solution. If the alkane or 1-alkanol is gaseous, the aqueous solution comprising the cell may be sparged with a gas comprising said gaseous alkane or 1-alkanol.

In case the alkane or 1-alkanol is solid, it may be solubilised using suitable organic solvents and then be added to the aqueous solution. Preferred organic solvents are biocompatible solvents, *i.e.* solvents that allow for the viability of the cell used and/or activity of the cytochrome P450 alkane hydroxylase from the CYP153 family chosen. The organic solvent is a fatty acid comprising 5 or more, more preferably 8 or more, most preferably 12 or more carbon atoms or derivatives thereof such as alkyl esters. The organic solvent is lauric acid methyl ester.

The cytochrome P450 alkane hydroxylase from the CYP153 family may be a recombinant hydroxylase. In a preferred embodiment, the term "recombinant" cytochrome P450 alkane hydroxylase from the CYP153 family, as used herein, means that the nucleic acid encoding the cytochrome P450 alkane hydroxylase from the CYP153 family is not an endogenous nucleic-acid of the organism used to express it, or is in fact not endogenous any wild type organism, but has been made using genetic engineering. The person skilled in the art is familiar with suitable plasmids, nucleic acid elements, for example promoter sequences and methods that may be used to make such plasmids. Standard molecular biology methods are described, for example, in Sambrook et al. (Molecular Cloning - A Laboratory Manual (1989) Cold Spring Harbor Laboratory Press).

Although a wild type strain expressing a cytochrome P450 alkane hydroxylase from the CYP153 family may in principle be used, it is preferred that the cytochrome P450 alkane hydroxylase from the CYP153 family is overexpressed in a recombinant strain. In a preferred embodiment, the term "overexpressed", as used herein, means that the concentration of the polypeptide in questions, compared to its concentration in the respective wild type cell, is elevated. The person skilled in the art is familiar with techniques that may be used for overexpressing a cytochrome P450 alkane hydroxylase from the CYP153 family, for example the use of the pET or pGEX system of plasmids.

The cytochrome P450 alkane hydroxylase from the CYP153 family may be a purified or isolated polypeptide. In a preferred embodiment, the term "purified", as used herein means that the polypeptide referred to as such is purer than it is at the time of its expression in a cell. The purity may be judged by polyacrylamide gel electrophoresis followed by Coomassie blue staining of the gel produced. In a preferred embodiment, the polypeptide is more than 50, 60, 70, 80, 90, 95 or 99 % pure, but it may in principle be used in any degree of purity, from crude cell lysate to 100 % pure polypeptide. The person skilled in the art is familiar with protein purification methods, for example affinity chromatography, ammonium sulphate precipitation and gel filtration chromatography.

The alkane or 1-alkanol is contacted with the cytochrome P450 alkane hydroxylase from the CYP153 family in an environment compatible with cytochrome P450 alkane hydroxylase activity. The person skilled in the art is familiar with standard parameters, such as ionic strength, temperature and the composition of suitable aqueous buffers that need to be considered with respect to enzyme activity and is capable of determining suitable conditions within the scope of routine experimentation. Suitable conditions for maintaining cytochrome P450 alkane hydroxylase activity are described, for example, in Scheps, D., Malca, H., Hoffmann, B., Nestl, B. M, und Hauer, B. (2011) Org. Biomol. Chem., 9, 6727.

It is essential that oxygen is present as long as the oxidation of the alkane or 1-alkanol catalysed by the cytochrome P450 alkane hydroxylase from the CYP153 family. Typically, the hydroxylase is in an aqueous solution, and oxygen is introduced by stirring the reaction vessel under aerobic conditions, *i.e.* in the presence of molecular oxygen (O₂). Alternatively, the solution may be sparged with pure molecular oxygen or gas mixtures comprising molecular oxygen, preferably in addition to inert gases such as nitrogen or argon. If the alkane or 1-alkanol to be oxidised is gaseous under the conditions contemplated, it may be part of such a mixture. In a preferred embodiment, the aqueous solution is in contact with air to provide oxygen.

In a preferred embodiment, molecular oxygen may be present at a partial pressure exceeding atmospheric pressure, preferably measured at room temperature (25 °C), preferably at more than 1.5, 2, 3, 4, 5, 6, 7, 8, 9, or 10 bar.

The teachings of the present invention may not only be carried out using biological macromolecules having the exact amino acid or nucleic acid sequences referred to in this application explicitly, for example by name or accession number, or implicitly, but also using variants of such sequences. The term "variant", as used herein, comprises amino acid or nucleic acid sequences, respectively, that are more than 70, 75, 80, 85, 90, 92, 94, 95, 96, 97, 98 or 99 % identical to the reference amino acid or nucleic acid sequence, wherein preferably amino acids other than those essential for the function, for example the catalytic activity of a protein, or the fold or structure of a molecule are deleted, substituted or replaced by insertions or essential amino acids are replaced in a conservative manner. The state of the art comprises algorithms that may be used to align two given nucleic acid or amino acid sequences and to calculate the degree of identity, see Arthur Lesk (2008), Introduction to bioinformatics, 3rd edition, Thompson et al., Nucleic Acids Research 22, 4637-4680, 1994, and Katoh et al., Genome Information, 16(1), 22-33, 2005. The term "variant" is used synonymously and interchangeably with the term "homologue". Such variants may be prepared by introducing deletions, insertions or substitutions in amino acid or nucleic acid sequences as well as fusions comprising such macromolecules or variants thereof. In a preferred embodiment, the term "variant", with regard to amino acid sequence, comprises, preferably in addition to the above sequence identity, amino acid sequences that comprise one or more conservative amino acid changes with respect to the respective reference or wild type sequence or comprises nucleic acid sequences encoding amino acid sequences that comprise one or more conservative amino acid changes. In a preferred embodiment, the term "variant" of an amino acid sequence or nucleic acid sequence comprises, preferably in addition to the above degree of sequence identity, any active portion and/or fragment of the amino acid sequence or nucleic acid sequence, respectively, or any nucleic acid sequence encoding an active portion and/or fragment of an amino acid sequence. In a preferred embodiment, the term "active portion", as used herein, refers to an amino acid sequence or a nucleic acid sequence, which is less than the full length amino acid sequence or codes for less than the full length amino acid sequence, respectively, wherein the amino acid sequence or the amino acid sequence encoded, respectively retains at least some of its essential biological activity. For example an active portion and/or fragment of a protease is capable of hydrolysing peptide bonds in polypeptides. In a preferred embodiment, the term "retains at least some of its essential biological activity", as used herein, means that the amino acid sequence in question has a biological activity exceeding and distinct from the background activity and the kinetic parameters characterising said activity, more specifically k_{cat} and K_{M}, are preferably within 3, more preferably 2, most preferably one order of magnitude of the values displayed by the reference molecule with respect to a specific substrate. In a preferred embodiment, the term "variant" of a nucleic acid comprises nucleic acids the complementary strand of which hybridises, preferably under stringent conditions, to the reference or wild type nucleic acid. Stringency of hybridisation reactions is readily determinable by one of ordinary skilled in the art, and in generally is an empirical calculation dependent on probe length, washing temperature and salt concentration. In general longer probes require higher temperatures for proper annealing, while shorter probes need lower temperatures. Hybridisation generally depends on the ability of denatured DNA to reanneal to complementary strands are present in an environment below their melting temperature. The higher the degree of desired homology between the probe and hybridisable sequence, the higher the relative temperature which may be used. As a result it follows that higher relative temperatures would tend to make the reaction conditions more stringent, while lower temperature less so. For additional details and explanation of stringency of hybridisation reactions, see F. M. Ausubel (1995), Current Protocols in Molecular Biology. John Wiley & Sons, Inc.,. Moreover, the person skilled take in the art may follow the instructions given in the manual "The DIG System Users Guide for Filter Hybridization", Boehringer Mannheim GmbH, Mannheim, Germany, 1993 and in Liebl et al. (International Journal of Systematic Bacteriology 41: 255-260 (1991) on how to identify DNA sequences by means of hybridisation. In a preferred embodiment, stringent conditions are applied for any hybridisation, i.e. hybridisation occurs only if the probe is 70 % or more identical to the target sequence. Probes having a lower degree of identity with respect to the target sequence may hybridise, but such hybrids are unstable and will be removed in a washing step under stringent conditions, for example lowering the concentration of salt to 2 x SSC or, optionally and subsequently, to 0,5 x SSC, while the temperature is, in order of increasing preference, approximately 50°C - 68°C, approximately 52°C - 68°C, approximately 54°C-68°C, approximately 56°C - 68°C, approximately 58°C - 68°C, approximately 60°C - 68°C, approximately 62°C - 68°C, approximately 64°C - 68°C, approximately 66°C - 68°C. In a particularly preferred embodiment, the temperature is approximately 64°C - 68°C or approximately 66°C - 68°C. It is possible to adjust the concentration of salt to 0.2 x SSC or even 0.1 x SSC. Polynucleotide fragments having a degree of identity with respect to the reference or wild type sequence of at least 70, 80, 90, 91, 92, 93, 94, 95, 96, 97, 98, 99 % may be isolated. In a preferred embodiment, the term "homologue" of a nucleic acid sequence, as used herein, refers to any nucleic acid sequence that encodes the same amino acid sequence as the reference nucleic acid sequence, in line with the degeneracy of the genetic code.

Alternatively, the cytochrome P450 alkane hydroxylase from the CYP153 family may be provided as a whole cell biocatalyst rather than an isolated polypeptide. In a preferred embodiment, the term "whole cell biocatalyst", as used herein, refers to a viable cell that expresses the cytochrome P450 alkane hydroxylase from the CYP153 family in a manner that allows contacting the latter with the alkane or 1-alkanol. In a preferred embodiment, cytochrome P450 alkane hydroxylase from the CYP153 family is located outside the cell at the outer cell membrane and so exposed to the aqueous solution surrounding the whole cell biocatalyst. Alternatively, the cytochrome P450 alkane hydroxylase from the CYP153 family is expressed inside the cell, preferably in the cytosol of the cell. The person skilled in the art is familiar with ways to routinely generate whole cell biocatalysts, see for example US 12/742,318.

The conversion of an alkane to a diol using a cytochrome P450 alkane hydroxylase from the CYP153 family expressed by a whole cell biocatalyst is limited not only by the activity of the hydroxylase expressed, but also by the amount of substrate imported into the cytosol of the whole cell biocatalyst used. In a preferred embodiment, the whole cell biocatalyst expresses, in addition to cytochrome P450 alkane hydroxylase from the CYP153 family, the polypeptide encoded by alkL from *Pseudomonas putida* (Access code CAB69081.1) or a variant thereof to facilitate import of alkane substrate. Examplary cells expressing AlkL and ways to make use of its capacity to import substrates into the cytosol of a biotechnologically relevant cell are described in WO2011/131420.

If the α,ω-diol is not the sought-after final product, it may be converted to other down stream products such as a diamine using reactions catalysed by synthetic catalysts or other enzymes. In case other enzymes are made use of, it is preferred that a whole cell biocatalyst is used that expresses, in addition to the cytochrome P450 alkane hydroxylase from the CYP153 family, other enzymes, for example one or more from the group comprising a transaminase, an alcohol dehydrogenase, an alanine dehydrogenase and the like. Examplary other enzymes are described in US 12/742,318.

If a whole cell biocatalyst expressing the cytochrome P450 alkane hydroxylase from the CYP153 family, and optionally in addition other enzymes, the whole cell biocatalyst may be further modified to increase the yield of the product, for example by deletion or inactivation of enzymes capable of degrading or metabolising said product. For example, if the sought-after product is an ester, one or more ester hydrolases, preferably those endogenous to the cell and encoded in the cell's genome, may be deleted or inactivated: Moreover, any oxidase capable of over oxidising one or two of the terminal hydroxyl groups of the sought-after α,ω-diol, may be deleted or inactivated.

The inventive teachings may be carried out using a wide range of cells as whole cell biocatalyst. In a preferred embodiment, the term "cell", as used herein, refers to any permanently unicellular cell comprising bacteria, archaea, fungi, algae and the like. In a preferred embodiment, the cell is a bacterial cell, more preferably one from the group comprising *Pseudomonas, Corynebacterium* and *Escherichia,* most preferably *Escherichia coli.* In another preferred embodiment, the cell is a lower eukaryote, more preferably a fungus from the group comprising *Saccharomyces, Candida, Picchia, Schizosaccharomyces* and *Yarrowia,* and is most preferably *Saccharomyces cerivisiae.* Throughout this application, the term "cell" is used synonymously and interchangeably with the term "microorganism".

The cell may be an isolated cell, in other words a pure culture of a single strain of cell, or may comprise a mixture of at least two strains. Biotechnologically relevant cells are commercially available, for example from the American Type Culture Collection (ATCC) or the German Collection of Microorganisms and Cell Cultures (DSMZ). Protocols for keeping and modifying microorganisms are available from the prior art, for example Sambrook/Fritsch/Maniatis (1989): Molecular cloning - A Laboratory Manual, Cold Spring Harbour Press, 2nd editi*on,* and Fuchs/Schlegel (2007), Allgemeine Mikrobiologie, 2008, Georg Thieme Verlag*.*

In a preferred embodiment, the whole cell biocatalyst used to carry out the inventive teachings has a reduced fatty acid degradation capacity. Degradation of fatty acids in microorganisms is achieved by a sequence of reactions and events as follows. First of all, fatty acids need to be transported across the cell membrane *via* a transport/acyl-activation mechanism involving at least one outer membrane protein and one inner membrane-associated protein which has fatty acid-CoA ligase activity, referred to in the case of *E. coli* as FadL and FadD, respectively. Inside the cell, the fatty acid to be degraded is subjected to the β-oxidation pathway. The first step
involves the conversion of acyl-CoA to enoyl-CoA through acyl-CoA dehydrogenase, referred as to FadE in the case of *E. coli.* The resulting enoyl-CoA is converted to 3-ketoacyl-CoA *via* 3-hydroxylacyl-CoA through hydration and oxidation, catalysed by enoyi-CoA hydratase/3-hydroxyacyl-CoA dehydrogenase, referred to as FadE in the case of *E. coli.* Finally, 3-ketoacyl-CoA thiolase, FadA in the case of *E. coli*, catalyses the cleavage of 3-ketoacyl-CoA, to give acetyl-CoA and an acyl-CoA shorten by two carbon atoms. In a preferred embodiment, the term "a microorganism having a reduced fatty acid degradation capacity", as used herein, refers to a microorganism having a reduced capability of taking up and/or degrading fatty acids, preferably those having at least eight carbon chains. The fatty acid degradation capacity of a microorganism may be reduce in various ways. In a preferred embodiment, the microorganism has, compared to its wild type, a reduced activity of an enzyme involved in the β-oxidation pathway, e. *g*. an enzyme that interacts directly with a fatty acid or a derivative thereof formed as part of the degradation said fatty acid *via* the β-oxidation pathway, preferably by recognizing the fatty acid or derivative thereof as a substrate and converting it to a metabolite formed as a part of the β-oxidation pathway and closer to an acetyl- CoA rather than the full length fatty acid. In a particularly preferred embodiment this includes a fatty acid importer, more specifically any component of the fatty acid import machinery. For example, the acyl-CoA dehydrogenase is an enzyme involved in the β-oxidation pathway as it interacts with fatty acid-CoA ester and converts the ester to enoyl CoA, which is closer to acetyl-CoA than to fatty acid-CoA ester. In a preferred embodiment, the term "enzyme involved in the fatty inter oxidation pathway", as used herein, comprises any polypeptide from the group comprising fatty acid importer and components thereof, acyl-CoA dehydrogenase, 2,4-dienoyl-CoA reductase, enoyl-CoA hydratase, 3-hydroxyacly-CoA dehydrogenase and 3-keton-acyl-CoA thiolase.

The inventive method comprises incubating butane, isobutane, butanol or isobutanol in an aqueous solution in the presence of cytochrome P450 alkane hydroxylase from the CYP153 family. This step may not only comprise temporarily contacting the alkane or 1-alkanol with the solution, but in fact incubating the alkane or 1-alkanol in the presence of the cytochrome P450 alkane hydroxylase from the CYP153 family sufficiently long to allow for an oxidation reaction to occur, for example for at least 3, 4, 5, 10 or 20 hours. The temperature chosen must be such that the inventive cell remains catalytically competent and/or metabolically active, for example 10 to 42 °C, preferably 30 to 40 °C, most preferably 32 to 38 °C in case the cell is an *E. coli* cell.

The alkane or 1-alkanol to be oxidised may be any alkane or 1-alkanol. The term "alkane", as used herein, refers to any compound represented by the formula CₙH₂ₙ₊₂, wherein n is or is more than 3, 4, 5, 6, 7, 8, 9, 10, 11, 12, 13, 14, 15, 16, 17, 18, 19, 20, 21, 22, 23, 24, 25, 26, 27, 28, 29, 30. The alkane is a cycloalkane represented by the formula CₙH₂ₙ, wherein n is or is more than 5, 6, 7, 8, 9, 10, 11, 12, 13, 14, 15, 16, 17, 18, 19, 20, 21, 22, 23, 24, 25, 26, 27, 28, 29, 30. The alkane or 1-alkanol is, at room temperature (25 °C) and atmospheric pressure, a gaseous alkane or 1-alkanol, for example isobutane. The term "1-alkanol", as used herein, refers to any compound represented by the formula CₙH₂ₙ₊₂O, wherein n is or is more than 3, 4, 5, 6, 7, 8, 9, 10, 11, 12, 13, 14, 15, 16, 17, 18, 19, 20, 21, 22, 23, 24, 25, 26, 27, 28, 29, 30. The term "1-alkanol", as used herein, refers to any cyclic compound represented by the formula CₙH₂ₙO, wherein n is or is more than 3, 4, 5, 6, 7, 8, 9, 10, 11, 12, 13, 14, 15, 16, 17, 18, 19, 20, 21, 22, 23, 24, 25, 26, 27, 28, 29, 30. The 1-alkanol is a primary aliphatic 1-alkanol.

A gaseous alkane or 1-alkanol is preferably provided at a partial pressure, preferably measured at room temperature (25 °C), exceeding atmospheric pressure, for example at more than 1.5, 2, 3, 4, 5, 6, 7, 8, 9 or 10 bar. The total combined pressure of all gaseous compounds present, for example oxygen, gaseous alkanes or 1-alkanols and nitrogen, may be more than 1.5, 2, 3, 4, 5, 6, 7, 8, 9 or 10 bar.

The term "an aqueous solution" comprises any solution comprising, as main solvent, water, that may be used to keep the whole cell biocatalyst expressing cytochrome P450 alkane hydroxylase from the CYP153 family, at least temporarily, in a metabolically active and/or viable state or the cytochrome P450 alkane hydroxylase from the CYP153 family catalytically competent and comprises, if such is necessary, any additional substrates. The person skilled in the art is familiar with numerous aqueous solutions, usually referred to as media, that may be used to grow and sustain cells, for example LB medium in the case of *E*. *coli.* The aqueous solution is kept under aerobic conditions. It is advantageous to use for growing cells to be used as whole cell biocatalysts a full medium owing to the increase in growth rate compared to minimal medium.

It is advantageous to use as an aqueous solution for carrying out the inventive method or use a simple buffer or minimal medium, *i.e.* a medium of reasonable simple composition that comprises only the minimal set of salts and nutrients indispensible for keeping the cell in a metabolically active and/or viable state, by contrast to complex mediums. For example, M9 medium may be used as a minimal medium. If the alkane or 1-alkanol to be oxidised has limited solubility in water, an organic solvent or detergent such as Tween or Triton may be added to the aqueous solution or a hydrophobic solvent may be used to solubilise the alkane or 1-alkanol to be oxidised. The person skilled in the art is familiar with the preparation of various aqueous and organic solutions.

The reaction may be run in a batch mode or in a continuous mode. The person skilled in the art is familiar with adequate fermenters and/or reaction vessels.
**Fig. 1** shows a GC/MS analysis of the products formed in Example 1, wherein the upper panel shows analysis of a regular sample, wherein the sample analysis shown in the lower panel is performed on a sample spiked with synthetic 1,4-butanediol as a standard.

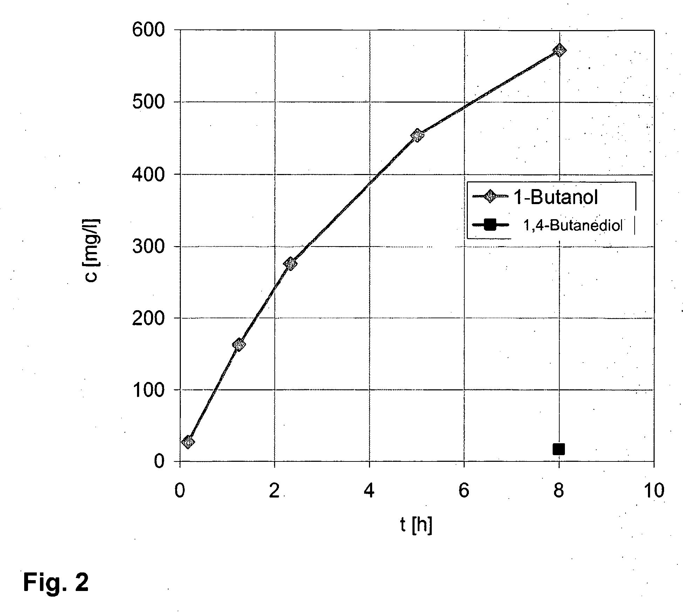

**Fig. 2** shows the production of butanol and 1,4-butanediol in Example 1 over time.
**Fig. 3** shows the production of 1,4-butanediol in Example 2 over time.

### Example 1: Oxidation of butane using E.coli W3110 pCOM10[Ab_Fd / CYP153-2 / FdOR / alkL] expressing the cytochrome P450 alkane hydroxylase from Alcanivorax borkumensis

Three 100-ml-flasks each comprising 25 ml LB-Medium supplemented with Kanamycin (composition: 5 g/l yeast extract, 10 g/l Pepton, 0,5 g/l NaCl, 50 mg/l Kanamycin sulfate) were inoculated using 100 ml each of glycerol cryoculture of *E.coli* W3110 pCOM10[Ab_Fd / CYP153-2 / FdOR / alkL] were incubated for 20 hours at 37 °C and 200 rpm.

Subsequently 25 ml each of a culture medium were transferred into 75 ml modified M9-Medium (composition: 15 g/l Glucose, 6.8 g/l Na₂PO₄, 3 g/l KH₂PO₄, 0,5 g/l NaCl, 2 g/l NH₄Cl, 15 g/l yeast extract, 0,49 g/l MgSO₄*7H₂O, 50 mg/l Kanamycin sulfate, 15 ml/l trace element solution US3. Composition of the trace element solution US3: 36.5 g/l 37 % hydrochloric acid, 1.91 MnCl₂*4H₂O, 1,87 g/l ZnSO₄*7H₂O, 0,84 g/l Na-EDTA*2H₂O, 0,3 g/l H₃BO₃, 0,25 g/l Na₂MoO₄*2H₂O, 4,7 g/l CaCl₂*2H₂O, 17,3 g/l FeSO₄*7H₂O, 0,15 g/l CuCl₂*2H₂O). The flasks were incubated at 37 °C and at 200 rpm for 2.5 hours. Subsequently the temperature was lowered to 25 °C. After 0.5 hours at 25 °C the culture was induced using 0.4 mM Dicyclopropylketone. Cultures were incubated for another 16 hours at 25 °C and 200 rpm.

Cultures were combined, transferred to 50 ml falcon tubes and spun down at 5500 g at 25 °C for 10 minutes. The supernatant was discarded, the pellets from 300 ml culture were resuspended in 50 ml conversion buffer at pH 7 (composition of the conversion buffer: 8 g/l (NH₄)H₂PO₄, 0,5 g/l NaCl, 0,49 g/l MgSO₄*7H₂O, 50 mg/l Kanamycin sulfate, 15 ml/l-trace element solution US3; pH adjusted to 7.0 using 25 % ammonia solution).

A 300 ml fermenter comprising 130 ml conversion buffer pH7 and approximately 3 drops of autoclaved antifoam reactant (Delamex) was set up. The fermenter was supplied with gas from a pressure gas flask (4 bar initial pressure) comprising a mixture consisting of 25 % n-butane and 75 % synthetic air via a sinterperlator having a pore size of 0.2 µm and a at flow rate of approximately 10 I_{N}/h. The fermenter was stirred in a water bath and the temperature of the fermenter was adjusted in a water bath to 30 °C and stirred using a magnetic stirrer at 900 rpm. pH value was maintained at 7.0 using 2.5 % aqueous ammonia. Glucose solution was fed continuously (glucose feed rate at 0.3 g/h). Air to be discarded was transferred to an ice-cooled wash bottle comprising 150 ml water.

The fermenter was inoculated using 50 ml of the resuspended preculture pellets.

The concentration of biomass in the fermenter, as indicated by optical density at 600 nm, was 16. 10 ml samples were removed from the fermenter and the wash bottle at various time points. The fermenter samples were spun down for 10 minutes at 10000 g and at room temperature and the supernatant was filtered using a 0.2 µm filter.

The analysis of 1-butanol was carried out chromatographically using HPLC-RID and an Agilent Technologies 1200 system. An Aminex HPX-87H column (300 mm x 7.8 mm) was used. The system was operated using 10 mM H₂SO₄ as running agent at a flow rate 0.6 ml/min and a column temperature of 40 °C. Standards of all substances to be analyzed were prepared in purest water and measured under identical conditions. The analysis was carried out by comparing retention time values.

Analysis of 1,4-butanediol was carried out qualitatively using GC-MS (TraceGC Ultra/DSQ II by ThermoScientific, capillary column: 30 m x 0.25 mm ZB-WAX df: 0.259 m, P013). Protein precipitation of the sample was carried out using cold acetone (sample/acetone 1 : 9). The supernatant spun down was subjected to GC-MS analysis. Substances were identified by comparing measured spectra and database spectra. 1,4-butanediol could be identified using its El-spectrum and could be assigned to the peak at a retention time of 19.88 min.

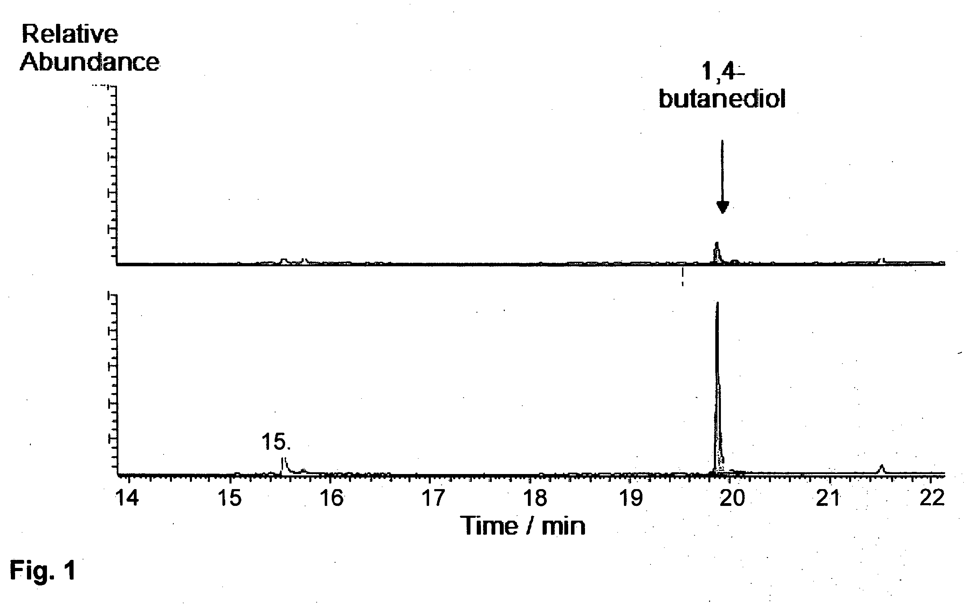

In order to confirm the identity the sample was spiked using 10 mg/l 1,4-butanediol. Fig. 1 shows the ion stream chromatography for the characteristic fragment ion m/z 71 of 1.4-butanediol. The concentration of 1,4-butanediol at 17 mg/l could be estimated by comparing the peak areas of spiked and non-spiked sample considering the dilution factor (VF = 10).

### Example 2:

### Oxidation of 1-butanol to 1,4-butanediol

The strain used in Example 1 could be shown to convert not only butane, but also 1-butanol to 1,4-butanediol.

Preseed culture: 1 L of LB medium (5 g/l yeast extract, 10 g/l peptone, 0,5 g/l NaCl, solved in 1 L water, autoclaved for 20 minutes at 121 °C) was prepared.

25 ml each of this solution were transferred into three 100 ml flasks with baffles, complemented with 25 µl of a kanamycin solution (50 g/l) sterilized by filtration and inoculated using 200 ml each of a glycerol cryoculture of *E.coli* W3110 pCOM10[Ab_Fd / CYP153-2 / FdOR / alkL]. These cultures were incubated at 37 °C and 200 rpm (amplitude 2.5 cm) for 20 hours.

Seed culture: 1 L of modified M9-medium was prepared, comprising 6,8 g/l Na₂HPO₄, 3 g/l KH₂PO₄, 15 g/l yeast extract, 0,5 g/l NaCl, 2 g/l NH₄Cl, 15 ml trace element solution US3 (1 L of the trace element solution US 3 comprises 36.5 g HCl 37 %, 1.91 g MnCl₂ x 4H₂O, 1.87 g ZnSO₄ x 7H₂O, 0.84 g sodium EDTA x 2H₂O, 0.3 g H₃BO₃, 0.25 g Na₂MOO₄ x 2H₂O, 4.7 g CaCl₂ x 2H₂O, 17.3 g FeSO₄ x 7 H₂O, 0.15 g CuCl₂, solved in 1 L water) 1 ml (MgSO₄ x 7H₂O)-solution (246,47 g/l), 30 ml glucose solution (500 g/l). 954 of the solution comprising Na₂HPO₄ to NH₄Cl were autoclaved, the other components were sterilized by filtration separately and added subsequently. The pH was 6.8.

3 x 175 ml of the modified M9-medium was transferred into 1000 ml shake flask with baffles, complemented with 75 µl of a Kanamycin solution (50 g/l) sterilized by filtration, inoculated with 25 ml preseed culture each and incubated for at 37 °C and 200 rpm (amplitude 2.5 cm). After 2.5 h at 37°C, the temperature was reduced to 25°C. After 0.5 h at 25°C the cultures were induced using 0.4 mM DCPK and incubated at 25°C for additional 16 h.

The cultures from the shake flasks were pooled in a sterile manner and spun down (6000g, 10 min, 25°C) in centrifuge flasks. The supernatant was discarded, and the pellets were resuspended in 30 ml 70 mM ammonium phosphate buffer at pH 7 (composition: 8 g/L (NH₄)H₂PO₄, 0.5 g/L NaCl, 0.49g/L MgSO₄ x 7H₂O, 15 ml trace element solution US3 and 50 µg/l Kanamycin, 5 % NH₄OH was used to adjust the pH).

### Biotransformation:

150 ml ammonium phosphate buffer comprising approximately 3 drops of autoclaved anti foam reagent (Delamex) was transferred into a sterile 300 ml fermenter. Air was introduced into the fermenter via a metal sinter perlator having a pore size of 0.2 µm at a flow rate of approximately 10 Nl/h. The temperature of the fermenter was maintained at 30 °C using a water bath, and their contents were stirred using a magnetic stirrer at 900 rpm. The outgoing air was passed through an ice-cooled washing bottle comprising 150 ml water.

The fermenter was inoculated using 30 ml of the resuspended biomass pellets via the sample-taking tube. pH was maintained at 7.0 using 2.5 % ammonia solution. A feed solution containing 30 g/l glucose and 50 g/l 1-butanol was fed continuously at a feed rate of 1.12 ml/h.

A 6 ml sample was removed from the fermenter after 0.2, 2, 4, 7, 22.5, 30.5 and 46.6 hours each. Samples were spun for 10 minutes at 10000 g and room temperature and the supernatant was filtered. Chromatographic analysis was carried out using GC-MS. Standards of 1,4-butanediol were prepared in ammonium phosphate buffer.

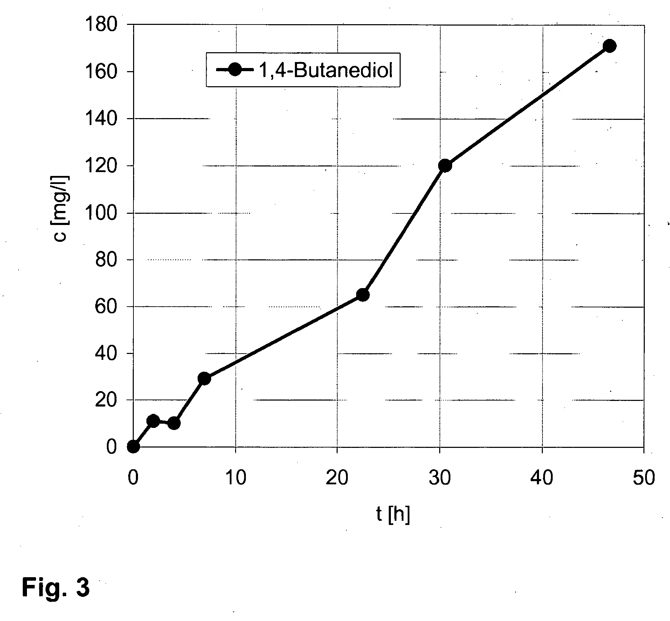

The concentration of 1,4-butanediol over time is depicted in **Fig. 3****.**

## Claims

1. A method comprising the steps
a) providing an alkane or 1-alkanol,
b) contacting said alkane or 1-alkanol in an aqueous solution with a cytochrome P450 alkane hydroxylase from the CYP153 family and oxygen for at least 3 hours,
wherein the alkane or 1-alkanol is converted to the corresponding α,ω-diol; and
the alkane or 1-alkanol is butane, isobutane, butanol or isobutanol.

2. The method according to claim 1, wherein the alkane or 1-alkanol is incubated in the presence of a cytochrome P450 alkane hydroxylase from the CYP153 family for at least 16hours.

3. The method according to either of claims 1 or 2, wherein, in addition to the cytochrome P450 alkane hydroxylase from the CYP153 family, a ferredoxin and a ferredoxin reductase is present, wherein both the ferredoxin and the ferredoxin reductase are capable of functionally interacting with said Cytochrome P450 alkane hydroxylase from the CYP153 family.

4. The method according to any of claims 1 to 3, wherein the cytochrome P450 alkane hydroxylase from the CYP153 family is the cytochrome P450 alkane hydroxylase from the CYP153 family from *Alcanivorax borkumensis* (Access codes YP_691921) or a variant thereof comprising an amino acid sequence that is more than 70 % identical to YP_691921 and that retains at least some of its essential biological activity, the ferredoxin is from *Alcanivorax borkumensis* or a variant thereof comprising an amino acid sequence that is more than 70 % identical to the ferredoxin from *Alcanivorax borkumensis* and that retains at least some of its essential biological activity and the Ferredoxin reductase is (Access code YP_691923) or a variant thereof comprising an amino acid sequence that is more than 70 % identical to YP_691923 and that retains at least some of its essential biological activity.

5. The method according to any one of claims 1 to 4, wherein the alkane is provided at a partial pressure of 1 to 50 bar.

6. The method according to any of claims 1 to 5, wherein at least one of the enzymes selected from the group comprising the cytochrome P450 alkane hydroxylase from the CYP153 family, ferredoxin and ferredoxin reductase are provided in the form of a whole cell catalyst expressing said enzyme or enzymes.

7. The method according to claim 6, wherein all of the enzymes selected from the group comprising the cytochrome P450 alkane hydroxylase from the CYP153 family, ferredoxin and ferredoxin reductase are provided in the form of a whole cell biocatalyst expressing said enzymes.

8. The method according to either of claims 6 or 7, wherein at least one of the enzymes selected from the group comprising the cytochrome P450 alkane hydroxylase from the CYP153 family, ferredoxin and ferredoxin reductase are recombinant and/or overexpressed.

9. The method according to any of claims 6 to 8, wherein the whole cell biocatalyst expresses a polypeptide from the AlkL family or a variant thereof comprising an amino acid sequence that is more than 70 % identical to the polypeptide from the AlkL family and that retains at least some of its essential biological activity.

10. The method according to any of claims 6 to 9, wherein the whole cell biocatalyst is an *E. coli.*

## Patentansprüche

1. Verfahren, umfassend die Schritte
a) Bereitstellen eines Alkans oder 1-Alkanols,
b) Inkontaktbringen des Alkans bzw. 1-Alkanols in einer wässrigen Lösung mit einer Cytochrom-P450-Alkan-Hydroxylase aus der CYP153-Familie und Sauerstoff über wenigstens 3 Stunden,
wobei das Alkan bzw. der 1-Alkanol in das entsprechende α,ω-Diol umgewandelt wird; und es sich bei dem Alkan bzw. 1-Alkanol um Butan, Isobutan, Butanol oder Isobutanol handelt.

2. Verfahren nach Anspruch 1, wobei das Alkan bzw. der 1-Alkanol in Gegenwart einer Cytochrom-P450-Alkan-Hydroxylase aus der CYP153-Familie wenigstens 16 Stunden inkubiert wird.

3. Verfahren nach Anspruch 1 oder 2, wobei zusätzlich zur Cytochrom-P450-Alkan-Hydroxylase aus der CYP153-Familie ein Ferredoxin und eine Ferredoxin-Reduktase vorhanden ist, wobei sowohl das Ferredoxin als auch die Ferredoxin-Reduktase zur funktionellen Wechselwirkung mit der Cytochrom-P450-Alkan-Hydroxylase aus der CYP153-Familie in der Lage sind.

4. Verfahren nach einem der Ansprüche 1 bis 3, wobei es sich bei der Cytochrom-P450-Alkan-Hydroxylase aus der CYP153-Familie um die Cytochrom-P450-Alkan-Hydroxylase aus der CYP153-Familie aus *Alcanivorax borkumensis* (Zugangscodes YP_691921) oder eine Variante davon, die eine Aminosäuresequenz umfasst, die zu mehr als 70% identisch mit YP_691921 ist und die wenigstens etwas von ihrer essentiellen biologischen Aktivität behält, handelt, das Ferredoxin aus *Alcanivorax borkumensis* stammt oder eine Variante davon ist, die eine Aminosäuresequenz umfasst, die zu mehr als 70% identisch mit dem Ferredoxin aus *Alcanivorax borkumensis* ist und die wenigstens etwas von seiner essentiellen biologischen Aktivität behält, und es sich bei der Ferredoxin-Reduktase um (Zugangscode YP_691923) oder eine Variante davon, die eine Aminosäuresequenz umfasst, die zu mehr als 70% identisch mit YP_691923 ist und die wenigstens etwas von ihrer essentiellen biologischen Aktivität behält, handelt.

5. Verfahren nach einem der Ansprüche 1 bis 4, wobei das Alkan mit einem Partialdruck von 1 bis 50 bar bereitgestellt wird.

6. Verfahren nach einem der Ansprüche 1 bis 5, wobei wenigstens eines der aus der die Cytochrom-P450-Alkan-Hydroxylase aus der CYP153-Familie, Ferredoxin und Ferredoxin-Reduktase umfassenden Gruppe ausgewählten Enzyme in Form eines das Enzym bzw. die Enzyme exprimierenden Ganzzellen-Katalysators bereitgestellt wird.

7. Verfahren nach Anspruch 6, wobei alle aus der die Cytochrom-P450-Alkan-Hydroxylase aus der CYP153-Familie, Ferredoxin und Ferredoxin-Reduktase umfassenden Gruppe ausgewählten Enzyme in Form eines die Enzyme exprimierenden Ganzzellen-Biokatalysators bereitgestellt werden.

8. Verfahren nach Anspruch 6 oder 7, wobei wenigstens eines der aus der die Cytochrom-P450-Alkan-Hydroxylase aus der CYP153-Familie, Ferredoxin und Ferredoxin-Reduktase umfassenden Gruppe ausgewählten Enzyme rekombinant und/oder überexprimiert ist.

9. Verfahren nach einem der Ansprüche 6 bis 8, wobei der Ganzzellen-Biokatalysator ein Polypeptid aus der AlkL-Familie oder eine Variante davon, die eine Aminosäuresequenz umfasst, die zu mehr als 70% identisch mit dem Polypeptid aus der AlkL-Familie ist und die wenigstens etwas von seiner essentiellen biologischen Aktivität behält, exprimiert.

10. Verfahren nach einem der Ansprüche 6 bis 9, wobei es sich bei dem Ganzzellen-Biokatalysator um ein *E. coli* handelt.

## Revendications

1. Procédé comprenant les étapes de
a) fourniture d'un alcane ou 1-alcanol,
b) mise en contact dudit alcane ou 1-alcanol dans une solution aqueuse avec une cytochrome P450 alcane hydroxylase de la famille CYP153 et de l'oxygène pendant au moins 3 heures,
dans lequel l'alcane ou 1-alcanol est converti en α,ω-diol correspondant ; et l'alcane ou 1-alcanol est le butane, l'isobutène, le butanol ou l'isobutanol.

2. Procédé selon la revendication 1, dans lequel l'alcane ou 1-alcanol est incubé en présence d'une cytochrome P450 alcane hydroxylase de la famille CYP153 pendant au moins 16 heures.

3. Procédé selon l'une des revendications 1 ou 2, dans lequel, en plus de la cytochrome P450 alcane hydroxylase de la famille CYP153, une ferrédoxine et une ferrédoxine réductase est présente, la ferrédoxine et la ferrédoxine réductase étant capables d'interagir fonctionnellement avec ladite cytochrome P450 alcane hydroxylase de la famille CYP153.

4. Procédé selon l'une quelconque des revendications 1 à 3, dans lequel la cytochrome P450 alcane hydroxylase de la famille CYP153 est la cytochrome P450 alcane hydroxylase de la famille CYP153 d'*Alcanivorax borkumensis* (codes d'accès YP_691921) ou un variant de celui-ci comprenant une séquence d'acides aminés qui est plus de 70 % identique à YP_691921 et qui conserve au moins une partie de son activité biologique essentielle, la ferrédoxine est d'*Alcanivorax borkumensis* ou un variant de celui-ci comprenant une séquence d'acides aminés qui est plus de 70 % identique à la ferrédoxine d'*Alcanivorax borkumensis* et qui conserve au moins une partie de son activité biologique essentielle et la ferrédoxine réductase est (code d'accès YP_691923) ou un variant de celui-ci comprenant une séquence d'acides aminés qui est plus de 70 % identique à YP_691923 et qui conserve au moins une partie de son activité biologique essentielle.

5. Procédé selon l'une quelconque des revendications 1 à 4, dans lequel l'alcane est fourni à une pression partielle de 1 à 50 bar.

6. Procédé selon l'une quelconque des revendications 1 à 5, dans lequel au moins une des enzymes choisies dans le groupe comprenant la cytochrome P450 alcane hydroxylase de la famille CYP153, la ferrédoxine et la ferrédoxine réductase est fournie sous la forme d'un catalyseur de cellule totale exprimant ladite enzyme ou lesdites enzymes.

7. Procédé selon la revendication 6, dans lequel toutes les enzymes choisies dans le groupe comprenant la cytochrome P450 alcane hydroxylase de la famille CYP153, la ferrédoxine et la ferrédoxine réductase sont fournies sous la forme d'un biocatalyseur de cellule entière exprimant lesdites enzymes.

8. Procédé selon une des revendications 6 ou 7, dans lequel au moins une des enzymes choisies dans le groupe comprenant la cytochrome P450 alcane hydroxylase de la famille CYP153, la ferrédoxine et la ferrédoxine réductase est recombinante et/ou surexprimée.

9. Procédé selon l'une quelconque des revendications 6 à 8, dans lequel le biocatalyseur de cellule entière exprime un polypeptide de la famille AlkL ou un variant de celui-ci comprenant une séquence d'acides aminés qui est plus de 70 % identique au polypeptide de la famille AlkL et qui conserve au moins une partie de son activité biologique essentielle.

10. Procédé selon l'une quelconque des revendications 6 à 9, dans lequel le biocatalyseur de cellule entière est *E. coli.*
